# EUROPEAN PATENT APPLICATION

(11) **EP 4 652 982 A1**
(43) Date of publication of application: **26.11.2025**
(21) Application number: 24744659.4
(22) Date of filing: 16.01.2024
(51) Int. Cl.: A61K 6/887, A61K 6/30, A61K 6/71, A61K 6/838

(54) **DENTAL RESTORATION MATERIAL COMPOSITION**

(30) Priority: 18.01.2023 JP 2023006117
(71) Applicant: GC R&D Corporation, Tokyo 174-8585 (JP)
(72) Inventor: TAKAHASHI, Makoto, Tokyo 174-8585 (JP); OHARA, Yuki, Tokyo 174-8585 (JP); KASAI, Yuki, Tokyo 174-8585 (JP)
(74) Representative: Müller-Boré & Partner Patentanwälte PartG mbB
(86) International application number: PCT/JP2024/001014
(87) International publication number: WO 2024/154738

(57) **Abstract**

A dental restoration material composition includes one or more acid-group-containing polymerizable monomers, one or more acid-group-free polymerizable monomers, and one or more inorganic fillers. An amount of the one or more inorganic fillers is 60 percent by mass or greater, and an amount of a water-insoluble polymerizable monomer that is free from an acid group and includes a benzene ring is 2.9 percent by mass or less.

## Description

### TECHNICAL FIELD

The present invention relates to dental restoration material compositions.

### BACKGROUND ART

In recent years, as dental restoration material compositions, such as dental composite resins, self-adhesive composite resins, in which an acid-group-containing polymerizable composition is blended, have been known (see, for example, Patent Document 1).

### CITATION LIST

### PATENT DOCUMENT

Patent Document 1: Japanese Patent No. 5882874

### SUMMARY OF THE INVENTION

### TECHNICAL PROBLEM

However, the adhesive dental restoration material compositions in the related art achieve relatively high initial adhesion, but have a problem of low bonding durability.

An object of the present invention is to provide a dental restoration material composition that excels in initial adhesion and bonding durability.

### SOLUTION TO THE PROBLEM

A dental restoration material composition according to one aspect of the present invention includes one or more acid-group-containing polymerizable monomers, one or more acid-group-free polymerizable monomers, and one or more inorganic fillers. An amount of the one or more inorganic fillers is 60 percent by mass or greater, and an amount of a water-insoluble polymerizable monomer that is free from an acid group and includes a benzene ring is 2.9 percent by mass or less.

### EFFECTS OF THE INVENTION

According to one aspect of the present invention, a dental restoration material composition that excels in initial adhesion and bonding durability can be provided.

### Detailed Description of the Embodiments

Embodiments of the present invention will be described in detail hereinafter.

### <Dental restoration material composition>

The dental restoration material composition according to the present embodiment includes one or more acid-group-containing polymerizable monomers, one or more acid-group-free polymerizable monomers, and one or more inorganic fillers. In the present specification, the dental restoration material composition encompasses a dental material for restoring a part of a tooth, which has been lost due to dental caries or the like.

The acid-group-containing polymerizable monomer is not particularly limited. For example, the acid-group-containing polymerizable monomer is an acid-group-containing (meth)acrylate. In the present specification, the (meth)acrylate denotes acrylate, methacrylate, or both acrylate and methacrylate.

Moreover, the acid-group-containing (meth)acrylate is not particularly limited. Examples of the acid-group-containing (meth)acrylate include a phosphoric acid-group-containing (meth)acrylate, a pyrophosphoric acid-group-containing (meth)acrylate, carboxyl group-containing (meth)acrylate, a thiophosphoric acid-group-containing (meth)acrylate, a sulfonic acid-group-containing (meth)acrylate, a phosphonic acid-group-containing (meth)acrylate, and the like.

Examples of the phosphoric acid-group-containing (meth)acrylate include: 2-(meth)acryloyloxyethyl dihydrogen phosphate, bis[2-(meth)acryloyloxyethyl] hydrogen phosphate, 2-(meth)acryloyloxyethyl phenyl hydrogen phosphate, 6-(meth)acryloyloxyhexyl dihydrogen phosphate, 6-(meth)acryloyloxyhexyl phenyl hydrogen phosphate, 10-(meth)acryloyloxydecyl dihydrogen phosphate, 1,3-di(meth)acryloylpropane-2-dihydrogen phosphate, 1,3-di(meth)acryloylpropane-2-phenyl hydrogen phosphate, bis[5-{2-(meth)acryloyloxyethoxycarbonyl}heptyl] hydrogen phosphate, etc.; acid chlorides of the foregoing; alkali metal salts of the foregoing; ammonium salts of the foregoing; and the like.

Examples of the pyrophosphoric acid-group-containing (meth)acrylate include: bis[2-(meth)acryloyloxyethyl] pyrophosphate, bis[4-(meth)acryloyloxybutyl] pyrophosphate, bis[6-(meth)acryloyloxyhexyl] pyrophosphate, bis[8-(meth)acryloyloxyoctyl] pyrophosphate, bis[10-(meth)acryloyloxydecyl] pyrophosphate, etc.; acid chlorides of the foregoing; alkali metal salts of the foregoing; ammonium salts of the foregoing; and the like.

Examples of the carboxyl group-containing (meth)acrylate include 4-(meth)acryloyloxyethyl trimellitate, 4-(meth)acryloyloxyethyltrimellitic anhydride, 4-(meth)acryloyloxydecyl trimellitate, 4-(meth)acryloyloxydecyltrimellitic anhydride, 11-(meth)acryloyloxy-1,1-undecanedicarboxylic acid, 1,4-di(meth)acryloyloxy pyromellitate, 2-(meth)acryloyloxyethyl maleate, 2-(meth)acryloyloxyethyl phthalate, 2-(meth)acryloyloxyethyl hexahydrophthalate, and the like.

Regarding the thiophosphoric acid-group-containing (meth)acrylate, examples of the thiophosphoric acid-group-containing (meth)acrylate include: 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 13-(meth)acryloyloxytridecyl dihydrogen thiophosphate, 14-(meth)acryloyloxytetradecyl dihydrogen thiophosphate, 15-(meth)acryloyloxypentadecyl dihydrogen thiophosphate, 16-(meth)acryloyloxyhexadecyl dihydrogen thiophosphate, 17-(meth)acryloyloxyheptadecyl dihydrogen thiophosphate, 18-(meth)acryloyloxyoctadecyl dihydrogen thiophosphate, 19-(meth)acryloyloxynonadecyl dihydrogen thiophosphate, 20-(meth)acryloyloxyicosyl dihydrogen thiophosphate, etc.; acid chlorides of the foregoing; alkali metal salts of the foregoing; ammonium salts of the foregoing; and the like.

Examples of the sulfonic acid-group-containing (meth)acrylate include 2-(meth)acrylamide-2-methylpropanesulfonic acid, styrene sulfonic acid, 2-sulfoethyl(meth)acrylate, and the like.

Examples of the phosphonic acid-group-containing (meth)acrylate include: 2-(meth)acryloyloxyethyl phenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl-3-phosphonoacetate, 10-(meth)acryloyloxydecyl-3-phosphonoacetate, etc.; acid chlorides of the foregoing; alkali metal salts of the foregoing; ammonium salts of the foregoing; and the like.

The acid-group-containing polymerizable monomer is preferably a phosphoric acid-group-containing (meth)acrylate. Among the above phosphoric acid-group-containing (meth)acrylates, 10-methacryloyloxydecyl dihydrogen phosphate (MDP) is more preferred in view of initial adhesion.

The above acid-group-containing polymerizable monomers may be used alone, or in combination.

An amount of the one or more acid-group-containing polymerizable monomers in the dental restoration material composition is not particularly limited. For example, the amount of the one or more acid-group-containing polymerizable monomers is from 0.5 percent by mass to 15 percent by mass, preferably from 1 percent by mass to 10 percent by mass, and more preferably from 2 percent by mass to 7 percent by mass, relative to 100 percent by mass of the dental restoration material composition. When the amount of the acid-group-containing polymerizable composition in the dental restoration material composition is from 0.5 percent by mass to 15 percent by mass, initial bonding adhesion of the dental restoration material composition is improved.

The acid-group-free polymerizable monomer is not particularly limited. For example, the acid-group-free polymerizable monomer is an acid-group-free (meth)acrylate, and more preferably an acid-group-free di(meth)acrylate.

The acid-group-free (meth)acrylate is not particularly limited. Examples of the acid-group-free (meth)acrylate include a (meth)acrylate including 8 ethylene oxide moieties or less per molecule, a urethane group-containing (meth)acrylate, and the like.

The (meth)acrylate including 8 ethylene oxide moieties or less per molecule is preferably a (meth)acrylate including 5 ethylene oxide moieties or less, and more preferably a (meth)acrylate including 4 ethylene oxide moieties or less. In the present specification, the ethylene oxide moieties include average repeating units.

Examples of the (meth)acrylate including 4 ethylene oxide moieties or less include triethylene glycol dimethacrylate (TEGDMA) and the like.

Examples of the urethane group-containing (meth)acrylate and the like include urethane dimethacrylate (UDMA) and the like.

The above acid-group-free (meth)acrylates may be used alone or in combination.

An amount of the acid-group-free polymerizable composition in the dental restoration material composition is not particularly limited. For example, the amount of the one or more acid-group-free polymerizable monomers is 5 percent by mass to 45 percent by mass, preferably from 10 percent by mass to 40 percent by mass, and more preferably from 15 percent by mass to 35 percent by mass, relative to 100 percent by mass of the dental restoration material composition. When the amount of the acid-group-free (meth)acrylate in the dental restoration material composition is 5 percent by mass to 45 percent by mass, bonding durability of the dental restoration material composition is improved, and mechanical strength of a cured body of the dental restoration material composition is improved.

In the case where triethylene glycol dimethacrylate (TEGDMA) is included as at least part of the one or more acid-group-free polymerizable monomers, an amount of TEGDMA is preferably 5 percent by mass or greater, more preferably 8 percent by mass or greater, and yet more preferably 10 percent by mass or greater, relative to 100 percent by mass of the dental restoration material composition.

In the case where the one or more acid-group-free polymerizable monomers include the (meth)acrylate including 8 ethylene oxide moieties or less per molecule (e.g., TEGDMA) and a polymerizable monomer other than the (meth)acrylate including 8 ethylene oxide moieties or less per molecule (e.g., the urethane group-containing (meth)acrylate) in the dental restoration material composition of the present embodiment, an amount of the (meth)acrylate including 8 ethylene oxide moieties or less per molecule is preferably greater than an amount of the polymerizable monomer other than the (meth)acrylate including 8 ethylene oxide moieties or less per molecule.

In the dental restoration material composition of the present embodiment, moreover, an amount of the one or more acid-group-free polymerizable monomers preferably includes the polymerizable monomer other than the acid-group-free (meth)acrylate, and is preferably greater than an amount of the one or more acid-group-containing polymerizable monomers.

The inorganic filler is not particularly limited. Examples of the inorganic filler include anhydrous silicic acid powders, fumed silica, alumina powders, glass powders (e.g., a barium glass powder and a fluoroaluminosilicate glass powder), and the like. Note that the above inorganic fillers may be subjected to a hydrophobic treatment with a surface treatment agent, such as a silane coupling agent.

The above inorganic fillers may be used alone or in combination.

An amount of the one or more inorganic fillers in the dental restoration material composition is, for example, preferably 60 percent by mass or greater, more preferably 65 percent by mass or greater, and yet more preferably 68 percent by mass or greater. Note that the upper limit of the amount of the one or more inorganic fillers in the dental restoration material composition is not particularly limited, but the upper limit is preferably 88 percent by mass or less, more preferably 85 percent by mass or less, and yet more preferably 83 percent by mass or less.

When the amount of the one or more inorganic fillers in the dental restoration material composition is 60 percent by mass or greater, mechanical strength of a cured body of the dental restoration material composition is improved. When the upper limit of the amount of the one or more inorganic fillers in the dental restoration material composition is 88 percent by mass or less, viscosity of a dental composition including the inorganic filler does not become too high so that reduction in operability of the dental restoration material composition can be minimized.

The dental restoration material composition of the present embodiment may further include a water-soluble polymerizable monomer that is free from an acid group (provided that, a below-described acrylamide-based polymerizable monomer is excluded). In the present specification, the term "water-soluble" refers to a characteristic of a substance that dissolves in water of 25°C at a concentration of 10 wt% or greater.

In this case, an amount of the water-soluble polymerizable monomer that is free from an acid group in the dental restoration material composition is 1.9 percent by mass or less, preferably 1 percent by mass or less, more preferably 0.4 percent by mass or less, and particularly preferably 0 percent by mass (not included), in view of inhibition in decline of bonding durability of the dental restoration material composition.

The water-soluble polymerizable monomer that is free from an acid group is not particularly limited. Examples of the water-soluble polymerizable monomer that is free from an acid group include a hydroxyl group-containing polymerizable monomer, a polymerizable monomer including 9 ethylene oxide moieties or more per molecule, and the like.

Specific examples of the hydroxyl group-containing polymerizable monomer include glycerol dimethacrylate (GDMA), hydroxyethyl methacrylate (HEMA), bisphenol A-glycidyl methacrylate (Bis-GMA), and the like.

Specific examples of the polymerizable monomer including 9 ethylene oxide moieties or more per molecule include polyethylene glycol #600 dimethacrylate (14G), ethoxylated bisphenol A dimethacrylate (m + n = 30; BPE-1300N), and the like.

The dental restoration material composition of the present embodiment may further include an acrylamide-based polymerizable monomer. In the present specification, the acrylamide-based polymerizable monomer encompasses a polymerizable monomer including, in a molecule, an acrylamide group or a methacrylamide group.

In this case, an amount of the acrylamide-based polymerizable monomer in the dental restoration material composition is preferably 1.9 percent by mass or less, more preferably 0.4 percent by mass or less, and particularly preferably 0 percent by mass (not included), in view of inhibition in decline of bonding durability of the dental restoration material composition.

The acrylamide-based polymerizable monomer is not particularly limited. Examples of the acrylamide-based polymerizable monomer include N,N-diethylacrylamide, N,N-hexamethylene bis(methacrylamide), and the like.

The dental restoration material composition of the present embodiment may further include a water-insoluble polymerizable monomer that is free from an acid group and includes a benzene ring. In the present specification, being water-insoluble encompasses a characteristic of a substance that is not dissolved in an amount of 0.1 wt% or greater in water of 25°C.

In this case, an amount of the water-insoluble polymerizable monomer that is free from an acid group and includes a benzene ring in the dental restoration material composition is 2.9 percent by mass or less, preferably 2 percent by mass or less, and more preferably 0 percent by mass (not included), in view of inhibition in decline of bonding durability of the dental restoration material composition.

The water-insoluble polymerizable monomer that is free from an acid group and includes a benzene ring is not particularly limited. Examples of the water-insoluble polymerizable monomer that is free from an acid group and includes a benzene ring include a compound of 2,2-bis(4-(meth)acryloyloxypolyethoxyphenyl)propane, in which an average number of moles of an ethoxy group added is 2.6 (BPE-100, D2.6E), and the like.

The dental restoration material composition of the present embodiment may further include a photopolymerization initiator, a photopolymerization accelerator, and a polymerization inhibitor.

The photopolymerization initiator is not particularly limited. Examples of the photopolymerization initiator include α-diketone compounds (e.g., camphorquinone (CQ)), acylphosphine oxide compounds (e.g., diphenyl(2,4,6-trimethylbenzoyl)phosphine oxide, diphenyl(2,4,6-trimethoxybenzoyl)phosphine oxide, diphenyl(2,6-dimethylbenzoyl)phosphine oxide, and diphenyl(2,6-dimethoxybenzoyl)phosphine oxide), benzyl ketal, diacetyl ketal, benzyl dimethyl ketal, benzyl diethyl ketal, benzyl bis(2-methoxyethyl)ketal, 4,4'-dimethyl(benzyldimethylketal), anthraquinone, 1-chloroanthraquinone, 2-chloroanthraquinone, 1,2-benzanthraquinone, 1-hydroxyanthraquinone, 1-methylanthraquinone, 2-ethylanthraquinone, 1-bromoanthraquinone, thioxanthone, 2-isopropylthioxanthone, 2-nitrothioxanthone, 2-methylthioxanthone, 2,4-dimethylthioxanthone, 2,4-diethylthioxanthone, 2,4-diisopropylthioxanthone, 2-chloro-7-trifluoromethylthioxanthone, thioxanthone-10,10-dioxide, thioxanthone-10-oxide, benzoin methyl ether, benzoin ethyl ether, benzoin isopropyl ether, benzoin isobutyl ether, benzophenone, bis(4-dimethylaminophenyl)ketone, 4,4'-bis(diethylamino)benzophenone, azide group-containing compounds, and the like. The above photopolymerization initiators may be used alone or in combination.

An amount of the photopolymerization initiator in the dental restoration material composition is not particularly limited. For example, the amount of the photopolymerization initiator is from 0.005 percent by mass to 5 percent by mass, and preferably from 0.01 percent by mass to 3 percent by mass, relative to 100 percent by mass of the dental restoration material composition. When the amount of the photopolymerization initiator in the dental restoration material composition is from 0.005 percent by mass to 5 percent by mass, adhesion of the dental restoration material composition is improved, and storage stability of the dental restoration material composition is improved.

The photopolymerization accelerator is not particularly limited. Examples of the photopolymerization accelerator include: tertiary amines, such as N,N-dimethyl-p-toluidine, triethanolamine, tolyldiethanolamine, methyl 4-dimethylaminobenzoate, ethyl 4-dimethylaminobenzoate (EPA), and isoamyl 4-dimethylaminobenzoate; barbituric acid and barbituric acid derivatives, such as 1,3-dimethylbarbituric acid, 1,3,5-trimethylbarbituric acid, 1,3,5-triethylbarbituric acid, 5-butylbarbituric acid, 1-benzyl-5-phenylbarbituric acid, and 1-cyclohexyl-5-ethylbarbituric acid; and the like. The above photopolymerization accelerators may be used alone or in combination.

An amount of the photopolymerization accelerator in the dental restoration material composition is not particularly limited. For example, the amount of the photopolymerization accelerator is preferably from 0.005 percent by mass to 5 percent by mass, and more preferably from 0.01 to 3 percent by mass, relative to 100 percent by mass of the dental restoration material composition. When the amount of the photopolymerization accelerator in the dental restoration material composition of the present embodiment is from 0.005 percent by mass to 5 percent by mass or less, polymerization efficiency of the dental restoration material composition is further improved.

The polymerization inhibitor is not particularly limited. Examples of the polymerization inhibitor include dibutylhydroxytoluene (BHT) (2,6-di-tert-butyl-p-cresol), 2,6-tert-butyl-2,4-xylenol, and the like. The above polymerization inhibitors may be used alone or in combination.

An amount of the polymerization inhibitor in the dental restoration material composition is not particularly limited. For example, the amount of the polymerization inhibitor is from 0.005 percent by mass to 3 percent by mass, and preferably from 0.01 percent by mass to 1 percent by mass, relative to 100 percent by mass of the dental restoration material composition.

Further, with regard to the dental restoration material composition of the present embodiment, a liquid component of the dental restoration material composition has a contact angle of less than 32°, preferably less than 30°, and more preferably less than 28°, with respect to dentin. In the present specification, the contact angle of the liquid component with respect to dentin encompasses an angle between dentin and the liquid component when a droplet of the liquid component is dropped on the dentin.

Further, with regard to the dental restoration material composition of the present embodiment, water sorption of a cured body of the dental restoration material composition is less than 40 µg/mm³, preferably less than 35 µg/mm³, and more preferably less than 30 µg/mm³. In the present specification, the water sorption of the cured body encompasses water sorption of a cured body in accordance with ISO 4049:2019 Dentistry-Polymer-based restorative materials, 7.12 Water sorption and solubility.

As described above, the dental restoration material composition of the present embodiment includes the one or more acid-group-containing polymerizable monomers, the one or more acid-group-free polymerizable monomers, and the one or more inorganic fillers. In addition, the amount of the one or more inorganic fillers is 60 percent by mass or greater, and the amount of a water-insoluble polymerizable monomer that is free from an acid group and includes a benzene ring is 2.9 percent by mass or less. Thus, the present embodiment can provide a dental restoration material composition that excels in both initial adhesion and bonding durability.

Since the amount of the water-soluble polymerizable monomer that is free from an acid group is 1.9 percent by mass or less, the dental restoration material composition of the present embodiment can inhibit decline of bonding durability.

Since the amount of the acrylamide-based polymerizable monomer is 1.9 percent by mass or less, the dental restoration material composition of the present embodiment can further inhibit decline of bonding durability.

Further, since the amount of the one or more acid-group-free polymerizable monomers is greater than the amount of the one or more acid-group-containing polymerizable monomers, the dental restoration material composition of the present embodiment can achieve both excellent initial adhesion and excellent bonding durability.

As described above, moreover, the one or more acid-group-free polymerizable monomer of the dental restoration material composition of the present embodiment include the (meth)acrylate including 8 ethylene oxide moieties or less per molecule. Therefore, bonding durability of the dental restoration material composition is improved, and mechanical strength of a cured body of the dental restoration material composition is improved.

Further, since the one or more acid-group-free polymerizable monomer further include the polymerizable monomer other than the (meth)acrylate including 8 ethylene oxide moieties or less per molecule, and the amount of the (meth)acrylate including 8 ethylene oxide moieties or less per molecule is greater than the amount of the polymerizable monomer other than the (meth)acrylate including 8 ethylene oxide moieties or less per molecule, the dental restoration material composition of the present embodiment can achieve both excellent initial adhesion and excellent bonding durability.

Further, as described above, the dental restoration material composition of the present embodiment includes the one or more acid-group-containing polymerizable monomers, the one or more acid-group-free polymerizable monomers, and the one or more inorganic fillers. In addition, the contact angle of the liquid component in the dental restoration material composition with respect to dentin is less than 32°, and water sorption of the cured body of the dental restoration material composition is less than 40 µg/mm³. Thus, the present embodiment can provide a dental restoration material composition that excels in initial adhesion and bonding durability.

### Examples

The present invention will be further described through Examples and Comparative Examples hereinafter. Note that various tests and evaluations were performed according to the following methods.

### <Preparation of dental restoration material composition>

Dental composite resins (Examples 1 to 9 and Comparative Examples 1 to 12) serving as dental restoration material compositions were prepared according to the corresponding compositions presented in Tables 1 and 2. Note that values in Tables 1 and 2 are presented by mass percentage (percent by mass).

Substances denoted by abbreviations or product names in Table 1 are as follows.
TEGDMA: triethylene glycol dimethacrylate
UDMA: urethane dimethacrylate
MDP: 10-methacryloyloxydecyl dihydrogen phosphate
HEMA: 2-hydroxyethyl methacrylate
N,N-Diethylacrylamide: N,N-diethylacrylamide
14G: polyethylene glycol #600 dimethacrylate
BPE-1300N: ethoxylated bisphenol A dimethacrylate
Bis-GMA: bisphenol A-glycidyl methacrylate
BPE-100: 2,2-bis(4-(meth)acryloyloxypolyethoxyphenyl)propane ethylene oxide (2.6) adduct
CQ: camphorquinone
EPA: ethyl 4-dimethylaminobenzoate
BHT: dibutylhydroxytoluene
AEROSIL: fumed silica (produced by NIPPON AEROSIL CO.,
LTD., AEROSIL is a registered trademark)

### <Initial bonding strength to dentin (initial adhesion to dentin)>

A lip side surface of an incisor of a bottom jaw of a cattle was polished with #400 silicon carbide paper (produced by Marumoto Struers) under a flow of water to expose a smooth surface, thereby producing a sample. After completing the polishing, the water on the surface of the sample was dried by air-blowing.

As a pre-treatment, a small amount of the dental composite resin prepared in each example was applied on the dried smooth surface of the sample, followed by agitating the applied dental composite resin for 10 seconds with an applicator. A plate-like mold (produced by ULTRADENT), in which a through-hole for material filing was formed at a center, was fixed to the sample. The through-hole had a diameter of 2.38 mm and a depth of 2.67 mm. Then, the smooth surface of the sample on which the dental composite resin had been applied was irradiated with light, which was applied over the mold and was transmitted via the through-hole of the mold, for 10 seconds using a dental polymerization visible-ray irradiator (product name: G-Light Prima II Plus, produced by GC).

Subsequently, the recess formed by the smooth surface of the sample and the through-hole was filled with the same dental composite resin as the above dental composite resin used in the small amount. The dental composite resin was irradiated with light over the mold for 10 seconds using the dental polymerization visible-ray irradiator in the same manner as above to harden the dental composite resin, thereby obtaining a hardened product. The mold was removed, thereby preparing a test piece. As the test piece, 5 test pieces were produced for each example, and the produced test pieces were left to stand in a thermostatic chamber of 37°C for 24 hours.

Thereafter, the test pieces were immersed in water of 37°C for 24 hours, followed by measuring a shear bond strength of each of the 5 test pieces by a universal tester (product name: Autograph, produced by Shimadzu Corporation) at a crosshead speed of 1.0 mm/min, and an arithmetic mean of the measured values was determined. The initial adhesion to dentin was evaluated based on the following criteria. The results are presented in Tables 1 and 2.

### [Evaluation Criteria]

Very Good: greater than 13 MPa
Good: greater than 11 MPa and 13 MPa or less
Not Good: 11 MPa or less

### <TC bonding strength to dentin (TC adhesion to dentin)>

Five test pieces were produced in total for each example in the same manner as the production of the test pieces obtained in the above test of the initial bonding strength to dentin. The produced test pieces were left to stand in a thermostatic chamber of 37°C for 24 hours, followed by immersing the test pieces in water of 37°C for 24 hours. Thereafter, a thermal cycling (TC) test was performed to evaluate bonding durability. As the TC test, a cycle of immersing in cold water of 5°C for 30 seconds, followed by immersing in warm water of 50°C for 30 seconds was repeated 10,000 times. A shear bond strength of each of the 5 test pieces was measured by a universal tester (product name: Autograph, produced by Shimadzu Corporation) at a crosshead speed of 1.0 mm/min, and an arithmetic mean of the measured values was determined. The TC adhesion to dentin was evaluated based on the following criteria. The results are presented in Tables 1 and 2.

### [Evaluation Criteria]

Very Good: greater than 13 MPa
Good: greater than 11 MPa and 13 MPa or less
Not Good: 11 MPa or less

### <Contact angle of liquid component with dentin (contact angle with dentin)>

A lip side surface of an incisor of a bottom jaw of a cattle was polished with #400 silicon carbide paper (produced by Marumoto Struers) under a flow of water to expose dentin, thereby producing a smooth surface of dentin as a sample. After completing the polishing, the water on the surface of the sample was dried by air-blowing.

The dried sample was set in an automatic contact angle meter (DM-501, produced by Kyowa Interface science Co., Ltd.), and a contact angle between the liquid component and the polished surface of the dentin of the extracted cattle tooth was measured. The amount of the liquid was 0.5 µL, and the contact angle was measured 1 minute after the droplet was deposited. The contact angle with dentin was evaluated based on the following criteria. The results are presented in Tables 1 and 2.

### [Evaluation Criteria]

Very Good: less than 28°
Good: 28° or greater and less than 32°
Not Good: 32° or greater

### <Water sorption of cured body of dental restoration material composition (water sorption of cured body)>

The water sorption of a cured body of each of Examples and Comparative Examples was measured according to ISO 4049:2019 Dentistry-Polymer-based restorative materials, 7.12 Water sorption and solubility. For each procedure of photocuring, light irradiation was performed for 10 seconds by a dental polymerization visible-ray irradiator (product name: G-Light Prima II Plus, produced by GC). The water sorption of the cured body of the dental restoration material composition was evaluated based on the following criteria. The results are presented in Tables 1 and 2.

### [Evaluation Criteria]

Very Good: less than 30 µg/mm³
Good: 30 µg/mm³ or greater and less than 40 µg/mm³
Not Good: 40 µg/mm³ or greater

### <Inorganic filler amount>

From the viewpoint that water sorption decreases as the amount of the inorganic filler increases, the amount of the inorganic filler was evaluated based on the following criteria. The results are presented in Tables 1 and 2.

### [Evaluation Criteria]

Very Good: 68 percent by mass or greater
Good: 65 percent by mass or greater and less than 68 percent by mass
Fair: 60 percent by mass or greater and less than 65 percent by mass
Not Good: less than 60 percent by mass

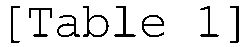

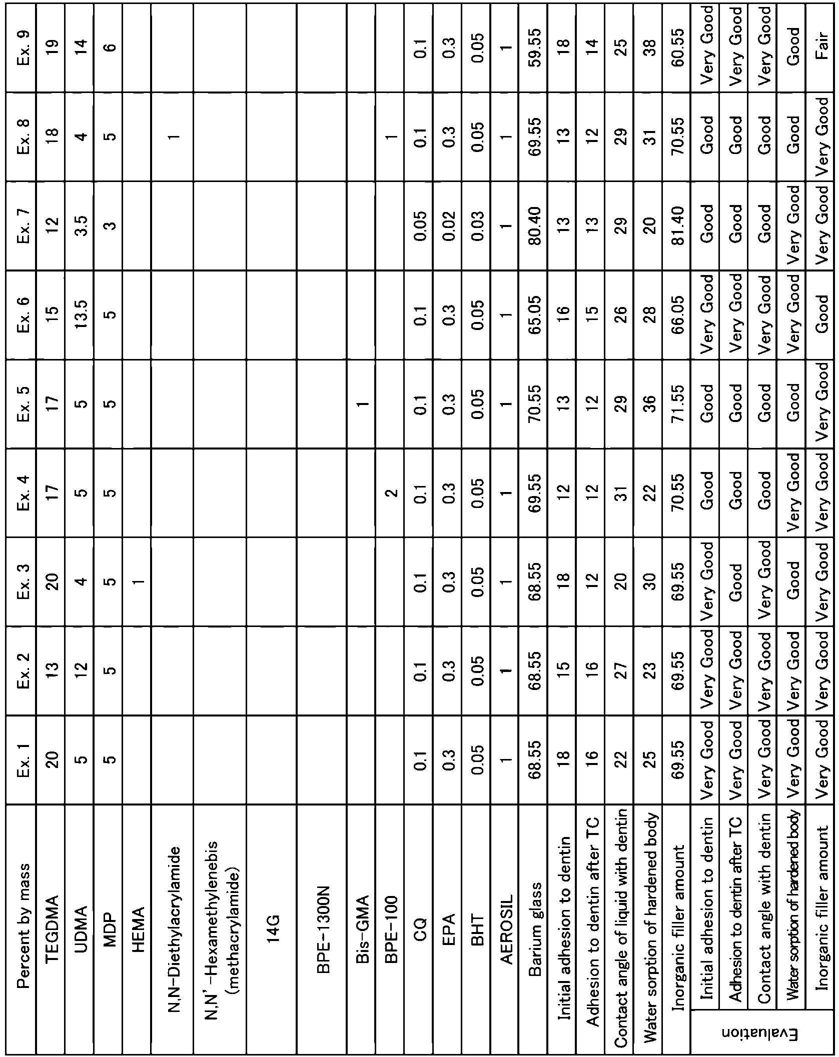

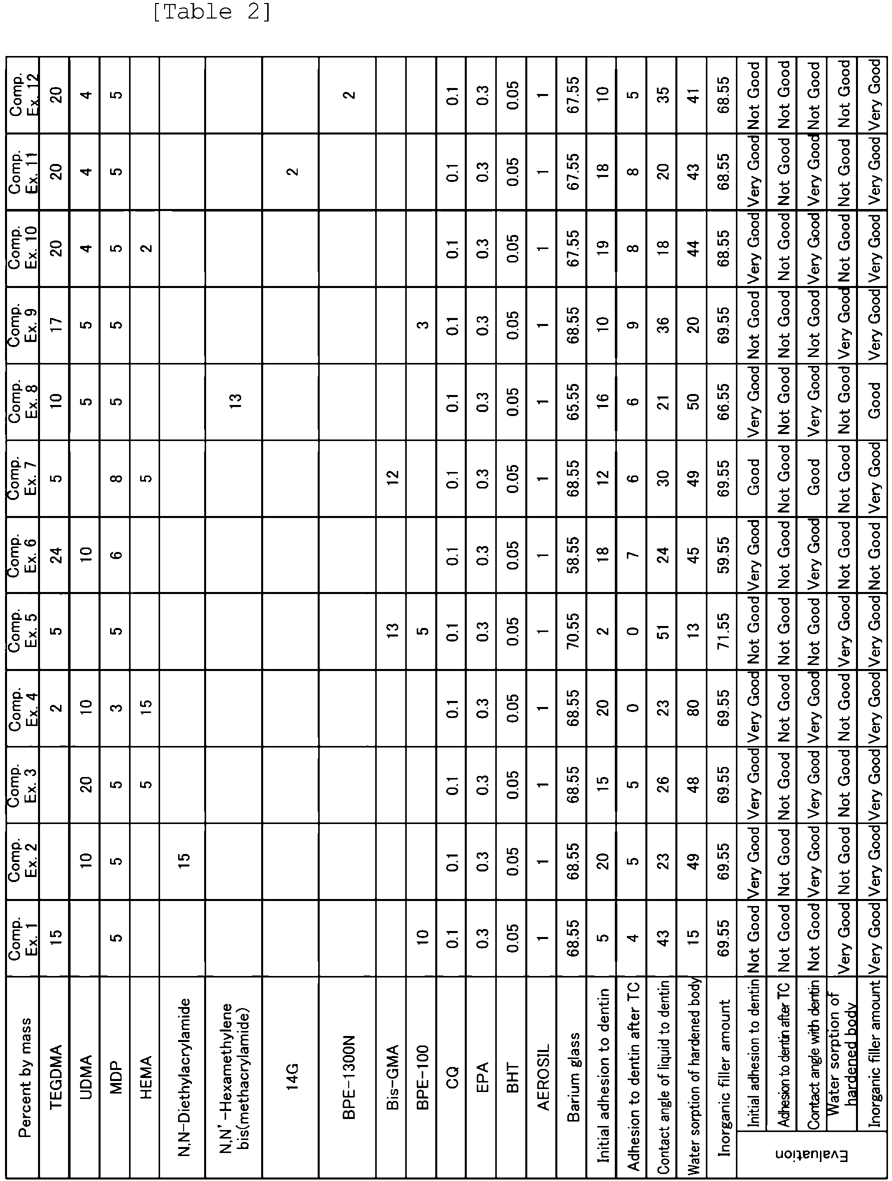

According to Table 1, the dental composite resins of Examples 1 to 9 had the results of Very Good or Good in the all of the initial adhesion to dentin, the TC adhesion to dentin, the contact angle with dentin, and the water sorption of the cured body, and had the results of Very Good, Good, or Fair in the inorganic filler amount.

According to Table 2, on the other hand, the dental composite resins of Comparative Examples 1 to 12 had the results of Not Good in at least one of the initial adhesion to dentin, the TC adhesion to dentin, the contact angle with dentin, the water sorption of the cured body, and the inorganic filler amount.

The embodiments described above include, for example, the following embodiments.

### (Clause 1)

A dental restoration material composition includes: one or more acid-group-containing polymerizable monomers; one or more acid-group-free polymerizable monomers; and one or more inorganic fillers. In the dental restoration material composition, an amount of the one or more inorganic fillers is 60 percent by mass or greater, and an amount of a water-insoluble polymerizable monomer that is free from an acid group and includes a benzene ring is 2.9 percent by mass or less.

### (Clause 2)

In the dental restoration material composition according to Clause 1, an amount of a water-soluble polymerizable monomer that is free from an acid group is 1.9 percent by mass or less.

### (Clause 3)

In the dental restoration material composition according to Clause 1 or 2, an amount of an acrylamide-based polymerizable monomer is 1.9 percent by mass or less.

### (Clause 4)

In the dental restoration material composition according to any one of Clauses 1 to 3, an amount of the one or more acid-group-free polymerizable monomers is greater than an amount of the one or more acid-group-containing polymerizable monomers.

### (Clause 5)

In the dental restoration material composition according to any one of Clauses 1 to 4, the one or more acid-group-free polymerizable monomers include a (meth)acrylate including 8 ethylene oxide moieties or less per molecule.

### (Clause 6)

In the dental restoration material composition according to any one of Clauses 1 to 5, the one or more acid-group-free polymerizable monomers include a polymerizable monomer other than the (meth)acrylate, and an amount of the (meth)acrylate is greater than an amount of the polymerizable monomer other than the (meth)acrylate.

### (Clause 7)

A dental restoration material composition includes: one or more acid-group-containing polymerizable monomers; one or more acid-group-free polymerizable monomers; and one or more inorganic fillers, where a contact angle of a liquid component in the dental restoration material composition is less than 32° with respect to dentin, and a water sorption of a cured body of the dental restoration material composition is less than 40 µg/mm³.

While embodiments of the present invention have been described above, the present invention is not limited to specific embodiments, and various modifications and variations are possible within the scope of the invention as claimed.

The present application is based on and claims priority to Japanese Patent Application No. 2023-006117, filed January 18, 2023, the contents of which are incorporated herein by reference.

## Claims

1. A dental restoration material composition comprising:
one or more acid-group-containing polymerizable monomers;
one or more acid-group-free polymerizable monomers; and
one or more inorganic fillers,
wherein an amount of the one or more inorganic fillers is 60 percent by mass or greater, and
an amount of a water-insoluble polymerizable monomer that is free from an acid group and includes a benzene ring is 2.9 percent by mass or less.

2. The dental restoration material composition according to claim 1,
wherein an amount of a water-soluble polymerizable monomer that is free from an acid group is 1.9 percent by mass or less.

3. The dental restoration material composition according to claim 1,
wherein an amount of an acrylamide-based polymerizable monomer is 1.9 percent by mass or less.

4. The dental restoration material composition according to claim 1,
wherein an amount of the one or more acid-group-free polymerizable monomers is greater than an amount of the one or more acid-group-containing polymerizable monomers.

5. The dental restoration material composition according to any one of claims 1 to 4,
wherein the one or more acid-group-free polymerizable monomers include a (meth)acrylate including 8 ethylene oxide moieties or less per molecule.

6. The dental restoration material composition according to claim 5,
wherein the one or more acid-group-free polymerizable monomers include a polymerizable monomer other than the (meth)acrylate, and
an amount of the (meth)acrylate is greater than an amount of the polymerizable monomer other than the (meth)acrylate.

7. A dental restoration material composition comprising:
one or more acid-group-containing polymerizable monomers;
one or more acid-group-free polymerizable monomers; and
one or more inorganic fillers,
wherein a contact angle of a liquid component in the dental restoration material composition is less than 32° with respect to dentin, and
a water sorption of a cured body of the dental restoration material composition is less than 40 µg/mm³.
